# EUROPEAN PATENT APPLICATION

(11) **EP 3 106 151 A1**
(43) Date of publication of application: **21.12.2016**
(21) Application number: 16175095.5
(22) Date of filing: 17.06.2016
(51) Int. Cl.: A61K 9/20, A61K 9/28, A61K 31/4365

(54) **PHARMACEUTICAL COMPOSITIONS OF PRASUGREL HYDROBROMIDE**

(30) Priority: 19.06.2015 TR 201507547
(71) Applicant: Sanovel Ilac Sanayi ve Ticaret A.S., 34460 Istanbul (TR)
(72) Inventor: TÜRKYILMAZ, Ali, 34460 Istanbul (TR); YILDIRIM, Ediz, 34460 Istanbul (TR); PALANTÖKEN, Arzu, 34460 Istanbul (TR); BAS, Oguz, 34460 Istanbul (TR); DEMIR, Bülent, 34460 Istanbul (TR); KARABULUT, Irem, 34460 Istanbul (TR)
(74) Representative: Sevinç, Erkan

(57) **Abstract**

The present invention relates to a pharmaceutical composition comprising prasugrel hydrobromide and a binder.

## Description

### Field of Invention

The present invention relates to a pharmaceutical composition comprising prasugrel hydrobromide and a binder.

### Background of Invention

Prasugrel is a member of the thienopyridine class. It is an inhibitor of platelet activation and aggregation, is indicated to reduce the rate of thrombotic cardiovascular events by means of P2Y12 and ADP receptors in patients with acute coronary syndrome.

There are different crystalline forms of Prasugrel, like Prasugrel hydrochloride, Prasugrel hydrobromide, Prasugrel phosphate, Prasugrel hydrogensulfate, Prasugrel nitrate and Prasugrel maleate. Only one of them is marketed under the trademark Effient® which contains Prasugrel hydrochloride in 5 or 10 mg dosages. It is initially administered in a dosage amount of 60 mg as a loading dose. It is used in preventing or treating thrombosis and cardiovascular diseases.

Chemical name of Prasugrel hydrobromide salt is 5-(2-Cyclopropyl-1-(2-fluorophenyl)-2-oxoethyl)-4,5,6,7-tetrahydrothieno 3,2-c]pyridin-2-yl acetate hydrobromide, with the chemical structure illustrated below in Formula I. Prasugrel hydrobromide is white and solid. There is no commercial product containing Prasugrel hydrobromide as the active substance yet.

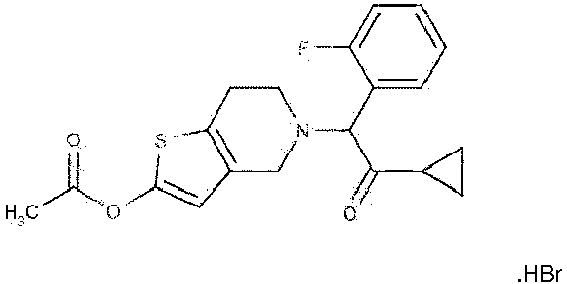

### Formula I. Prasugrel hydrobromide

The patent application EP2722037 A1 discloses prasugrel free base, a method for preparing a stable tablet formulation of prasugrel free base.

The patent application EP1298132 discloses hydrochloride salt of prasugrel, a method for preparing this salt, and its use for thrombosis and embolism.

The patent application EP1728794 discloses maleate salt prasugrel, a method for preparing this salt, and its use for thrombosis and embolism.

The patent application WO2011127300 discloses crystalline forms of prasugrel salts.

The patent application WO2011004392 discloses a crystalline form of prasugrel hydrobromide, the preparation method and application.

The patent application WO2013091595 discloses a pharmaceutical composition containing as the active substance prasugrel hydrobromide form C.

The patent application WO2006138317 discloses a dosage administration required for the loading dose and following doses of prasugrel.

The patent application WO2008073759 discloses a tablet or capsule formulation containing prasugrel, packaged in an air- and moisture-impermeable blister under a gas with positive fluidity pressure in order to reduce the amount of oxygen and humidity generated during packaging.

Stability-related problems occur in many active agents, including prasugrel, under the influence of environmental and physical conditions. However, prasugrel is an active agent that is highly-susceptible to air and humidity. When prasugrel is exposed to air and humidity, it degrades structurally and develops chemical behavioral changes. As a result of this, two main problems emerge. The first problem is that the stability of the products developed is not at a desired level and the shelf life thereof is shortened. The second problem is that prasugrel reacts with the excipients employed in developing formulations containing prasugrel. This fact causes impurities to occur in the formulation and leads to incorporation of undesired components into the formulation. In addition to these, measures should be taken while preparing, packaging, and storing finished dosage forms comprising prasugrel.

As a consequence, a need rises for formulations of prasugrel and a process for preparing such formulations that overcome the problems mentioned above.

However, as a result of studies made, it was surprisingly found that pharmaceutical formulations comprising prasugrel hydrobromide is more resistant against environmental and physical conditions as compared to pharmaceutical formulations comprising other forms, particularly the hydrochloride and maleate salts, of prasugrel.

The present invention offers a prasugrel formulation that is resistant against environmental and physical conditions, and have an improved stability under production and storage conditions. Also, this invention prevents the impurity formation in the formulation and presents improved dissolution profile. Moreover, the formulation is stable under production, packaging and storage conditions.

Therefore, a novelty is required in the art of prasugrel formulations used for preventing and treating thrombosis and cardiovascular diseases, which would overcome the aforesaid drawbacks. Advantages and embodiments of the present invention will become apparent from the following description.

### Detailed Description of Invention

In the main embodiment, the present invention relates a pharmaceutical composition comprising prasugrel hydrobromide and a binder.

In one embodiment, the pharmaceutical composition of this present invention reduces the rate of thrombotic cardiovascular events (including stent thrombosis) in patients with acute coronary syndrome and prevents serious or life-threatening problems with the heart and blood vessels in patients.

In one embodiment, prasugrel used in this present invention is prasugrel hydrobromide.

According to this embodiment, prasugrel hydrobromide is present in an amount of between 0.1 - 30.0 %, preferably 0.5 - 20.0 % and more preferably 1 - 10.0 % by weight of total formulation.

According to this embodiment, prasugrel hydrobromide is present in an amount of between 1 mg and 50mg, preferably between 5mg and 30mg and more preferably it is in an amount of between 5mg and 20mg.

In one embodiment, said binder used in this present invention is selected from the group comprising copovidone (Kollidon VA 64), copolyvidone, polyvinylpyrrolidone (PVP), , povidone K30, carnauba wax, hydroxypropyl methyl cellulose (HPMC), pullulan, polymethacrylate, glyceryl behenate, hydroxypropyl cellulose (HPC), carboxymethyl cellulose (CMC), methyl cellulose (MC), hydroxyethyl cellulose, sodium carboxymethyl cellulose (Na CMC), carboxymethyl cellulose calcium, ethyl cellulose and other cellulose derivatives, polymetacrylates, polyethylene oxide, polyvinyl alcohol, polycarbophil, polyvinyl acetate and their copolymers, gelatin, starch, xanthan gum, guar gum, alginate, carrageen, kollagen, agar, pectin, hyaluronic acid, carbomer, cellulose acetate phthalate, hydroxypropyl starch, hydroxyethyl methyl cellulose, polaxomer, polyethylene glycol (PEG), sugars, glycose syrups, natural gums, tragacanth gum, polyacrylamide, aluminum hydroxide, benthonite, laponite, setostearyl alcohol, polyoxyethylene-alkyl ethers, acacia mucilage, polydextrose and mixtures thereof.

In this embodiment, binder used in this present invention is copovidone.

Copovidone is a white to yellowish-white amorphous powder. It is typically spray-dried with a relatively fine particle size. It has a slight odor and a faint taste. It provides good adhesion, elasticity, and hardness, and can be used as a moisture barrier. Kollidon VA 64 has a spherical structure, with a high proportion of damaged spheres. In this invention, the shell-like structure reduces flowability, but the damaged spheres cover a greater surface area of the filler particles, increasing the efficacy of its use as a dry binder.

According to this embodiment, copovidone (Kollidon VA 64) is present in an amount of between 0.1 - 15.0 %, preferably 0.5 - 10.0 % and more preferably 1.0 - 5.0 % by weight of total formulation.

According to one embodiment, the ratios of prasugrel hydrobromide and copovidone as a binder used in this present invention ensure the required effective doses for the therapy and make it possible to ensure the stability and improved dissolution profile.

In one embodiment, it has been found that in certain ratio of prasugrel hydrobromide to copovidone as a binder which is between 0.05 - 30.0(w/w), preferably 0.1 - 20.0 (w/w) and more preferably 0.2- 10.0 (w/w) helps the composition easily processed into a stable dosage form.

According to one embodiment, the pharmaceutical composition is in the form of tablets coated tablets, trilayer tablets, bilayer tablets, multilayer tablets, orally disintegrating tablets, mini tablets, capsules, pellet, sugar pellet, buccal tablets, sublingual tablets, effervescent tablets, immediate release tablets, modified release tablets, film-coated tablets, gastric disintegrating tablets, pills, hard or soft gelatin capsules, oral granules, powders, coated bead systems, granules, microspheres, tablet in tablets, inlay tablets, dragees, sachets, orally administrable films, ion exchange resin systems and thereof.

In one embodiment, pharmaceutical composition of this present invention is in the form of a tablet or a coated tablet.

According to one embodiment, pharmaceutical composition of this present invention is in the form of a coated tablet comprising prasugrel hydrobromide and copovidone.

According to one embodiment, pharmaceutical combination of this present invention further comprise pharmaceutical excipients selected from the group comprising lubricants, glidants, disintegrants, diluents, taste-masking coatings and mixtures thereof.

In one embodiment, lubricant used in this present invention is selected from the group comprising sodium lauryl sulphate, sodium stearyl fumarate, magnesium stearate, zinc stearate, calcium stearate, mineral oil, talc, polyethylene glycol, glyceryl monostearate, glyceryl palmitostearate, magnesium lauryl sulphate, fumaric acid, zinc stearate, stearic acid, hydrogenated natural oils, silica, paraffin and mixtures thereof.

In this embodiment, lubricants used in this present invention are sodium lauryl sulphate and sodium stearyl fumarate.

Sodium stearyl fumarate is supplied in a pure form and is often of value when the less pure stearate-type lubricants are unsuitable owing to chemical incompatibility. Sodium stearyl fumarate is less hydrophobic than magnesium stearate or stearic acid and has a less retardant effect on tablet dissolution than magnesium stearate. So sodium stearyl fumarate is chosen for this present invention. (Handbook of Pharmaceutical Excipients, fifth edition, Rowe, Raymond C., Sheskey, Paul J., Owen, Sian C., pages 705-707*).*

In this pharmaceutical composition of the present invention, it is objected to overcome the technical problems which are shown above by using sodium stearyl fumarate instead of magnesium stearate as a lubricant wherein the specific percentage amount of sodium stearyl fumarate in a formulation according to the present in an amount of between 0.1 - 10.0 %, preferably 0.5 - 7.0 % and more preferably 0.5 - 5.0 % by weight of total formulation. According to this embodiment, sodium lauryl sulphate is present in an amount of between 0.1 - 10.0 %, preferably 0.5 - 7.0 % and more preferably 1.0 - 5.0 % by weight of total formulation.

According to one embodiment, the ratios of between lubricants used in this present invention ensure the required effective doses for the therapy and make it possible to ensure the stability and improved dissolution profile.

In one embodiment, it has been found that in certain ratio of sodium stearyl fumarate to sodium lauryl sulphate which is between 0.01 - 10.0(w/w), preferably 0.05 - 5.0 (w/w) and more preferably 0.1-1.0 (w/w).

In one embodiment, glidant used in this present invention is selected from the group comprising colloidal silicon dioxide (Aerosil) (Aerosil 200), talc, aluminum silicate, powdered cellulose, calcium phosphate tribasic, hydrophobic colloidal silica, magnesium oxide, magnesium trisilicate, magnesium silicate and mixtures thereof.

In this embodiment, glidant used in this present invention is colloidal silicon dioxide.

Colloidal silicon dioxide has small particle size and large specific surface area give it desirable flow characteristics that are exploited to improve the flow properties of dry powder of this invention especially during tableting process.

According to this embodiment, colloidal silicon dioxide is present in an amount of between 0.01 - 10.0 %, preferably 0.05 - 5.0 % and more preferably 0.1 - 2 % by weight of total formulation.

According to one embodiment, the ratios of between glidant to lubricant used in this present invention ensure the required effective doses for the therapy and make it possible to ensure the compressibility, stability and improved dissolution profile.

In one embodiment, it has been found that in certain ratio of colloidal silicon dioxide to sodium lauryl sulphate and sodium stearyl fumarate which is between 0.005 - 10.0(w/w), preferably 0.01 - 5.0 (w/w) and more preferably 0.05-1.0 (w/w).

Suitable disintegrant is selected from the group comprising croscarmellose sodium, sodium carbonate, HPC (hydroxylpropyl cellulose), microcristalline cellulose, crospovidon (crosslinked polyvinylpyrrolidone), copovidon, polycarbophil, low-substitue poloxamer, sodium starch glycolate, starch, alginic acid and alginates, ion-exchange resins, magnesium aluminum silica, sodium dodecyl sulphate, sodium carboxy methyl cellulose, carboxy methyl cellulose calcium, docusate sodium, guar gum, polyacrylin potasium, sodium alginate, sodium glysin carbonate, sodium lauryl sulphate and mixtures thereof.

Suitable diluent is selected from the group comprising lactose monohydrate, mannitol, lactose, corn starch dibasic calcium phosphate, xylitol, microcrystalline cellulose (MCC), spray-dried lactose, sorbitol, sucrose, trehalose, isomalt, starch, calcium phosphate anhydrate, calcium phosphate dihydrate, calcium phosphate trihydrate, tribasic calcium phosphate, calcium carbonate, calcium sulfate, carboxymethyl cellulose calcium, powdered cellulose, cellulose acetate, pregelatinized starch, sodium carbonate, sodium bicarbonate, isomalt, maltodextrine, dextrose, calcium carbonate, sugars, magnesium carbonate and mixtures thereof.

Suitable moisture protective coatings is selected from the group comprising hydroxypropylmethyl cellulose, hydroxypropyl cellulose, polyvinyl alcohol (PVA), polyethylene glycol, talc, polyvinyl alcohol-polyethylene glycol copolymers (Kollicoat IR), butyl methacrylate and methyl methacrylate (Eudragit E 100) (Poly(butyl methacrylate-co-(2-demethylaminoeethyl)methacrylate-co-methyl methacrylate)), ethylcellulose dispersions (Surelease), Kerry-HPC, polyvinylprolidone, polyvinylprolidone-vinyl acetate copolymer (PVP-VA) and all kinds of OpadryTM, as well as pigments, dyes, titanium dioxide, iron oxide or polymethylmetacrylate copolymers (Eudragit) and mixtures thereof.

In this present invention, to provide a pharmaceutical composition comprising prasugrel hydrobromide and a binder, these tablet formulations have been designed, comprising the followings:

**Tablet-1:**

| | |
|---|---|
| a) | 1.0 - 10.0 % by weight of prasugrel hydrobromide, |
| b) | 1.0 - 5.0 % by weight of copovidone, |
| c) | 30.0 - 55.0 % by weight of microcrystalline cellulose, |
| d) | 45.0 - 55.0 % by weight of lactose monohydrate, |
| e) | 1.0 - 10.0 % by weight of croscarmellose sodium, |
| f) | 25.0 - 40.0 % by weight of mannitol, |
| g) | 1.0 - 5.0 % by weight of sodium lauryl sulphate, |
| h) | 0.1 - 2.0 % by weight of colloidal silicon dioxide, |
| i) | 0.5 - 5.0 % by weight of sodium stearyl fumarate, |
| j) | 1.0 - 5.0 % by weight of coating: |
| | a. 30.0 - 50.0 % by weight of hydroxypropylmethyl cellulose, |
| | b. 15.0 - 35.0 % by weight of hydroxypropyl cellulose, |
| | c. 10.0 - 30.0 % by weight of titanium dioxide, |
| | d. 5.0 - 25.0 % by weight of talc. |

**Tablet-2:**

| | |
|---|---|
| a) | 1.0 - 10.0 % by weight of prasugrel hydrobromide, |
| b) | 1.0 - 5.0 % by weight of copovidone, |
| c) | 45.0 - 55.0 % by weight of lactose monohydrate, |
| d) | 30.0 - 55.0 % by weight of microcrystalline cellulose, |
| e) | 1.0 - 10.0 % by weight of croscarmellose sodium, |
| f) | 25.0 - 40.0 % by weight of mannitol, |
| g) | 1.0 - 5.0 % by weight of sodium lauryl sulphate, |
| h) | 0.1 - 2.0 % by weight of colloidal silicon dioxide, |
| i) | 0.5 - 5.0 % by weight of sodium stearyl fumarate, |
| j) | 1.0 - 5.0 % by weight of coating: |
| | a. 25.0 - 50.0 % by weight of hydroxypropylmethyl cellulose, |
| | b. 10.0 - 30.0 % by weight of polyvinyl alcohol, |
| | c. 20.0 - 40.0 % by weight of titanium dioxide, |
| | d. 1.0 - 15.0 % by weight of polyethylene glycol, |
| | e. 1.0-15.0 % by weight of talc. |

Accordingly, the pharmaceutical formulation according to the present invention comprising prasugrel hydrobromide as an active agent is unexpectedly resistant against environmental and physical conditions and both shows a substantially improved stability under production and storage conditions. Using prasugrel hydrobromide as the active agent and copovidone as a binder bring the stability of the formulation to a desired level. Additionally, above-mentioned process for preparing the formulation, helps the stability of finished dosage forms of the formulation under production, packaging, storage conditions to be increased and dissolution profile to be improved.

The present invention is used for treating thrombosis and cardiovascular diseases.

These formulations can be used in the content of medicaments which is used for treating thrombosis and cardiovascular diseases.

**Example 1: Coated Tablet**

| **Ingredients** | **Amount (%)** |
|---|---|
| Prasugrel hydrobromide | 1.0 - 10.0 |
| Copovidone (Kollidon VA 64) | 1.0 - 5.0 |
| Microcrystalline cellulose | 30.0 - 55.0 |
| Lactose monohydrate | 45.0 - 55.0 |
| Croscarmellose sodium | 1.0 - 10.0 |
| Mannitol | 25.0 - 40.0 |
| Sodium lauryl sulphate | 1.0 - 5.0 |
| Colloidal silicon dioxide (Aerosil 200) | 0.1 - 2.0 |
| Sodium stearyl fumarate | 0.5 - 5.0 |
| Coating | 1.0 - 5.0 |

| **Coating ingredients** | **Amount (%)** |
|---|---|
| Hydroxypropylmethyl cellulose | 30.0 - 50.0 |
| Hydroxypropyl cellulose | 15.0 - 35.0 |
| Titanium dioxide | 10.0 - 30.0 |
| Talc | 5.0 - 25.0 |

The pharmaceutical formulation mentioned above is prepared as following:
- Prasugrel hydrobromide, Copovidone (Kollidon VA 64), microcrystalline cellulose, lactose monohydrate, croscarmellose sodium, mannitol and sodium lauryl sulphate are sieved and mixed.
- Then, mixture is granulated with water. Granules are sieved and mixed then dried.
- Then, colloidal silicon dioxide (Aerosil 200) and sodium stearyl fumarate are added to
- mixture and mixed again. Mixture is pressed into tablets.
- Hydroxypropylmethyl cellulose, hydroxypropyl cellulose, titanium dioxide and talc are mixed and %15-22 coating solution is obtained. The tablets are coated with this mixture.

**Example 2: Coated Tablet**

| **Ingredients** | **Amount (%)** |
|---|---|
| Prasugrel hydrobromide | 1.0 - 10.0 |
| Copovidone (Kollidon VA 64) | 1.0 - 5.0 |
| Lactose monohydrate | 45.0 - 55.0 |
| Microcrystalline cellulose | 30.0 - 55.0 |
| Croscarmellose sodium | 1.0 - 10.0 |
| Mannitol | 25.0 - 40.0 |
| Sodium lauryl sulphate | 1.0 - 5.0 |
| Colloidal silicon dioxide (Aerosil 200) | 0.1 - 2.0 |
| Sodium stearyl fumarate | 0.5 - 5.0 |
| Coating | 1.0 - 5.0 |

| **Coating ingredients** | **Amount (%)** |
|---|---|
| Hydroxypropylmethyl cellulose | 25.0 - 50.0 |
| Polyvinyl alcohol | 10.0 - 30.0 |
| Titanium dioxide | 20.0 - 40.0 |
| Polyethylene glycol | 1.0 - 15.0 |
| Talc | 1.0 - 15.0 |

The pharmaceutical formulation mentioned above is prepared as following:
- Prasugrel hydrobromide, Copovidone (Kollidon VA 64), lactose monohydrate, microcrystalline cellulose, croscarmellose sodium, mannitol and sodium lauryl sulphate are sieved and mixed.
- Then, mixture is granulated with water. Granules are sieved and mixed then dried.
- Then, colloidal silicon dioxide (Aerosil 200) and sodium stearyl fumarate are added to
- mixture and mixed again. Mixture is pressed into tablets.
- Hydroxypropylmethyl cellulose, polyvinyl alcohol, titanium dioxide, polyethylene glycol and talc are mixed and %15-22 coating solution is obtained. The tablets are coated with this mixture.

## Claims

1. A pharmaceutical composition comprising, prasugrel hydrobromide and a binder.

2. The pharmaceutical composition according to claim 1, wherein prasugrel hydrobromide is present in an amount of between 0.1 - 30.0 %, preferably 0.5 - 20.0 % and more preferably 1 - 10.0 % by weight of total formulation.

3. The pharmaceutical composition according to claim 1 or 2, prasugrel hydrobromide is present in an amount of between 1 mg and 50mg, preferably between 5mg and 30mg and more preferably it is in an amount of between 5mg and 20mg.

4. The pharmaceutical composition according to claim 1, wherein said binder is selected from the group comprising copovidone, copolyvidone, polyvinylpyrrolidone (PVP), povidone K30, carnauba wax, hydroxypropyl methyl cellulose (HPMC), pullulan, polymethacrylate, glyceryl behenate, hydroxypropyl cellulose (HPC), carboxymethyl cellulose (CMC), methyl cellulose (MC), hydroxyethyl cellulose, sodium carboxymethyl cellulose (Na CMC), carboxymethyl cellulose calcium, ethyl cellulose and other cellulose derivatives, polymetacrylates, polyethylene oxide, polyvinyl alcohol, polycarbophil, polyvinyl acetate and their copolymers, gelatin, starch, xanthan gum, guar gum, alginate, carrageen, kollagen, agar, pectin, hyaluronic acid, carbomer, cellulose acetate phthalate, hydroxypropyl starch, hydroxyethyl methyl cellulose, polaxomer, polyethylene glycol (PEG), sugars, glycose syrups, natural gums, tragacanth gum, polyacrylamide, aluminum hydroxide, benthonite, laponite, setostearyl alcohol, polyoxyethylene-alkyl ethers, acacia mucilage, polydextrose and mixtures thereof.

5. The pharmaceutical composition according to claim 1 to 4, wherein said binder is copovidone.

6. The pharmaceutical composition according to claim 5, copovidone is present in an amount of between 0.1 - 15.0 %, preferably 0.5 - 10.0 % and more preferably 1.0 - 5.0 % by weight of total formulation.

7. The pharmaceutical composition according to claim 1 to 6, wherein the ratio of prasugrel hydrobromide to copovidone as a binder which is between 0.05 - 30.0(w/w), preferably 0.1 - 20.0 (w/w) and more preferably 0.2- 10.0 (w/w).

8. The pharmaceutical composition according to claim 1, wherein the composition is in the form of tablets coated tablets, trilayer tablets, bilayer tablets, multilayer tablets, orally disintegrating tablets, mini tablets, capsules, pellet, sugar pellet, buccal tablets, sublingual tablets, effervescent tablets, immediate release tablets, modified release tablets, film-coated tablets, gastric disintegrating tablets, pills, hard or soft gelatin capsules, oral granules, powders, coated bead systems, granules, microspheres, tablet in tablets, inlay tablets, dragees, sachets, orally administrable films, ion exchange resin systems and thereof.

9. The pharmaceutical composition according to claim 8, the composition is in the form of a tablet or a coated tablet.

10. The pharmaceutical composition according to claim 5 to 9, the composition is in the form of a coated tablet comprising prasugrel hydrobromide and copovidone.

11. The pharmaceutical composition according to any preceding claims, further comprising pharmaceutical excipients selected from the group comprising lubricants, glidants, disintegrants, diluents, taste-masking coatings and mixtures thereof.

12. The pharmaceutical composition according to any preceding claims, tablet formulation comprising;
| | |
|---|---|
| a) | 1.0 - 10.0 % by weight of prasugrel hydrobromide, |
| b) | 1.0 - 5.0 % by weight of copovidone, |
| c) | 30.0 - 55.0 % by weight of microcrystalline cellulose, |
| d) | 45.0 - 55.0 % by weight of lactose monohydrate, |
| e) | 1.0 - 10.0 % by weight of croscarmellose sodium, |
| f) | 25.0 - 40.0 % by weight of mannitol, |
| g) | 1.0 - 5.0 % by weight of sodium lauryl sulphate, |
| h) | 0.1 - 2.0 % by weight of colloidal silicon dioxide, |
| i) | 0.5 - 5.0 % by weight of sodium stearyl fumarate, |
| j) | 1.0 - 5.0 % by weight of coating: |
| | a. 30.0 - 50.0 % by weight of hydroxypropylmethyl cellulose, |
| b. | 15.0 - 35.0 % by weight of hydroxypropyl cellulose, |
| c. | 10.0 - 30.0 % by weight of titanium dioxide, |
| d. | 5.0 - 25.0 % by weight of talc. |

13. The pharmaceutical composition according to any preceding claims, tablet formulation comprising;
| | |
|---|---|
| a) | 1.0 - 10.0 % by weight of prasugrel hydrobromide, |
| b) | 1.0 - 5.0 % by weight of copovidone, |
| c) | 45.0 - 55.0 % by weight of lactose monohydrate, |
| d) | 30.0 - 55.0 % by weight of microcrystalline cellulose, |
| e) | 1.0 - 10.0 % by weight of croscarmellose sodium, |
| f) | 25.0 - 40.0 % by weight of mannitol, |
| g) | 1.0 - 5.0 % by weight of sodium lauryl sulphate, |
| h) | 0.1 - 2.0 % by weight of colloidal silicon dioxide, |
| i) | 0.5 - 5.0 % by weight of sodium stearyl fumarate, |
| j) | 1.0 - 5.0 % by weight of coating: |
| | a. 25.0 - 50.0 % by weight of hydroxypropylmethyl cellulose, |
| | b. 10.0 - 30.0 % by weight of polyvinyl alcohol, |
| | c. 20.0 - 40.0 % by weight of titanium dioxide, |
| | d. 1.0 - 15.0 % by weight of polyethylene glycol, |
| | e. 1.0 - 15.0 % by weight of talc. |

14. The pharmaceutical composition according to any preceding claims, is used for treating thrombosis and cardiovascular diseases.
